(19) Europäisches Patentamt
European Patent Office
Office européen des brevets

(11) Veröffentlichungsnummer: **0 246 646 B1**

(12) # EUROPÄISCHE PATENTSCHRIFT

(45) Veröffentlichungstag der Patentschrift: **09.01.91 Patentblatt 91/02**

(51) Int. Cl.⁵: **C07C 45/74**, C07C 47/277, C07D 319/06

(21) Anmeldenummer: **87107391.2**

(22) Anmeldetag: **21.05.87**

(54) **Verfahren zur Herstellung von 4-Acetalen des Butendial-(1,4) und neue Acetale des Butendial-(1,4).**

(30) Priorität: **23.05.86 DE 3617409**

(43) Veröffentlichungstag der Anmeldung: **25.11.87 Patentblatt 87/48**

(45) Bekanntmachung des Hinweises auf die Patenterteilung: **09.01.91 Patentblatt 91/02**

(84) Benannte Vertragsstaaten: **BE CH DE FR GB IT LI**

(56) Entgegenhaltungen:
**DE-A- 2 513 999**
**FR-A- 2 189 359**
**"Liebigs Ann. Chem.", 1976, Seiten 2194-2205; J. PAUST et al.: "Neue C5-Bausteine für Terpensynthesen, I. Monoacetale von 2-Methyl-2-buten-1,4-dial"**

(73) Patentinhaber: **BASF Aktiengesellschaft**
**Carl-Bosch-Strasse 38**
**D-6700 Ludwigshafen (DE)**

(72) Erfinder: **Merger, Franz, Dr.**
**Max-Slevogt-Strasse 25**
**D-6710 Frankenthal (DE)**
Erfinder: **Fischer, Rolf, Dr.**
**Bergstrasse 98**
**D-6900 Heidelberg (DE)**
Erfinder: **Horler, Hans, Dr.**
**Hainweg 20**
**D-6100 Darmstadt (DE)**
Erfinder: **Frank, Juergen, Dr.**
**Hirschbrunnenweg 82**
**D-6830 Schwetzingen (DE)**

EP 0 246 646 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Anmeldung betrifft ein Verfahren zur Herstellung von in 2-Stellung substituierten 4-Acetalen des Butendial-(1,4) durch Umsetzung von Glyoxalmonoacetalen mit Aldehyden, die α-ständige Methylengruppen aufweisen und neue Acetale des Butendial-(1,4).

4-Acetale des 2-Methyl-fumardialdehyds sind wertvolle Bausteine für die Synthese von Terpenen mit biologischer und pharmakologischer Wirksamkeit. Für ihre Herstellung sind schon mehrere Verfahren vorgeschlagen worden. So ist aus den DE-OS 23 57 752 und 23 57 810 bekannt, daß sich Acetale des 3-Methyl-2-butenals mit Selendioxid zu den entsprechenden 4-Acetalen des 2-Methyl-fumardialdehyds oxidieren lassen. In der DE-OS 22 25 612 wird ein Verfahren zur Herstellung cyclischer 4-Acetale des 2-Methyl-fumardialdehyds beschrieben, bei dem man die entsprechenden Acetale des 3-Methyl-4-hydroxi-2-butenals mit schwefelsaurer Chromsäurelösung oxidiert.

In der EP-PS 9 752 wird die Umsetzung von cyclischen Acetalen des 3-Methyl-3-butenals mit Nitrosierungsmitteln, wie Nitrosylchlorid oder Salpetrigsäureestern in Gegenwart von Methanol und Salzsäure beschrieben. Man erhält dabei 2-Chlor-2-methyl-butan-1,4-dial-bisacetale, aus denen man durch Abspaltung von Chlorwasserstoff mit Basen Bisacetale des 2-Methyl-2-buten-1,4-dials gewinnt, die sich mit verdünnten wäßrigen Säuren selektiv zu 4-Acetalen des 2-Methylfumardialdehyds hydrolysierenlassen.

Bei all diesen Verfahren werden Oxidationsmittel verwendet, die teuer und/oder toxisch sind und deren Reduktionsprodukte Entsorgungsprobleme schaffen. Dies ist besonders bei Selendioxid, Chromsäure, Nitrosylchlorid oder Salpetrigsäureestern der Fall. Verwendet man Sauerstoff oder Sauerstoff übertragende Verbindungen (Wasserstoffperoxid, Percarbonsäuren oder organische Hydroperoxide), so erfordern diese Oxidationsmittel einen hohen Sicherheitsaufwand. Außerdem sind die benötigten Ausgangsverbindungen meist nur in vielstufigen Synthesen zu erhalten.

Bei der in der DE-OS 2 513 999 beschriebenen Synthese setzt man Glyoxalmonoacetale mit Grignardverbindungen zu Acetalen des 2-Hydroxi-3-butenalsum, acetyliert diese anschließend zu den entsprechenden Acetalen des 2-Acetoxi-3-butenals und erhält dann durch Hydroformylierung in Gegenwart von Rhodium-Verbindungen ein Gemisch aus Acetalen des 3- und 4-Formyl-2-acetoxi-butanals, von denen lediglich die 3-Formylverbindung nach Abspaltung von Essigsäure 4-Acetale des 2-Methylfumardialdehyds liefert.

Es wurde nun gefunden, daß man 4-Acetale des Butendial-(1,4) der Formel

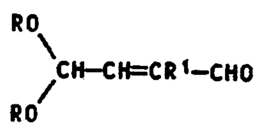

I

in der R für einen Alkyl-, Alkenyl-, Cycloalkyl- oder Aralkylrest mit 1 bis 12 C-Atomen steht, der Alkoxygruppen enthalten kann, oder beide R zusammen einen Alkylen- oder Alkenylenrest mit 2 bis 10 C-Atomen, der Alkoxigruppen enthalten kann, bedeuten und $R^1$ einen Alkyl-, Alkenyl- oder Alkinylrest mit 1 bis 12 C-Atomen, der durch cycloaliphatische, aromatische oder heterocyclische Reste oder durch Hydroxi-, Ether-, Thioether-, Acyl-, Alkylamino-, Carboxi- oder Carbalkoxygruppen substituiert sein kann, einen gegebenenfalls substituierten Arylrest oder eine Alkoxi-, Alkylthio- oder Acyloxygruppe bedeutet, besonders vorteilhaft dadurch erhält, daß man Glyoxalmonoacetale der Formel

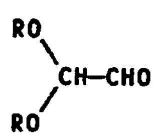

II,

in der die Reste R die oben genannte Bedeutung haben, mit Aldehyden der Formel

$$R^1\text{-}CH_2\text{-}CHO \quad \text{III}$$

in der $R^1$ die obengenannte Bedeutung hat, bei Temperaturen von 20 bis 150°C umsetzt.

Das vorteilhafte Ergebnis des erfindungsgemäßen Verfahrens, das es erlaubt, Glyoxalmonoacetale in einem Reaktionsschritt mit Propionaldehyd in 4-Acetale des 2-Methylfumar-dials zu überführen und das es

darüberhinaus ermöglicht, auch andere Aldehyde mit α-ständigen Methylengruppen zu in 2-Stellung substituierten 4-Acetalen des Fumardials umzusetzen, konnte aufgrund der obengenannten technischen Kenntnisse nicht erwartet werden.

In den Glyoxalmonoacetalen der Formel II steht R z.B. für einen Alkyl-, Alkenyl-, Aralkyl- oder Cycloalkylrest mit 1 bis 12, vorzugsweise 1 bis 8 C-Atomen, wie für Methyl, Ethyl, Propyl, Allyl, Butyl, Butenyl, Isobutyl, Methallyl, Benzyl, Cyclopentyl, Cyclohexyl oder Cyclooctyl. Die beiden Reste R können auch gemeinsam einen geradkettigen oder verzweigten Alkylenrest mit 2 bis 8, vorzugsweise 2 bis 5 C-Atomen, der Alkoxigruppen, wie Methoxi- oder Ethoxigruppen tragen kann, bedeuten. Das sind z.B. Reste der Formeln $-(CH_2)_2-$, $-(CH_2)_3-$, $-(CH_2)_4-$, $-CH_2-C(CH_3)_2-CH_2-$, $-CH_2-CH(CH_3)-CH_2-$, $-(CH_2)_2-CH(CH_3)-CH_2-$, $-CH_2-CH(CH_3)-$, $-CH_2-C(CH_3)(CH_3OCH_2-CH_2)-CH_2-$, $-CH_2-CH(OCH_3)-CH_2-$ und $-CH_2-CH(CH_2-OCH_3)-$.

Im einzelnen seien z.B. genannt die Monoacetale des Glyoxals mit Methanol, Ethanol, Propanol, Allylalkohol, (iso-)Butanol, Methallylalkohol, Methoxiethanol, Ethoxiethanol, Glykol, 1,2- und 1,3-Propylenglykol, Butandiol-(1,3), 2-Methyl-propandiol-(1,3), (2,2-Dialkyl-propandiole-(1,3), 2-Methyl-2-methoximethyl-propandiol-(1,3), Ethylmethoximethylpropandiol-(1,3), Butandiol-(1,4), 2-Methylbutandiol-(1,4) und 2-Ethylbutandiol-(1,4). Besonders gut geeignet ist das Monoacetal des Glyoxals mit Neopentylglykol : 2-Formyl-5,5-dimethyl-dioxan-(1,3).

In den Aldehyden der Formel III bedeutet $R^1$ z.B. einen geradkettigen oder verzweigten Alkyl-, Alkenyl- oder Alkinylrest mit 1 bis 12, vorzugsweise 1 bis 8 C-Atomen, der cycloaliphatische, aromatische oder heterocyclische Reste, wie Phenyl- oder Pyridyl-Reste, und Hydroxi-, Alkoxi-, Thioether-, Acetoxy-, Alkylamino-, Carboxi- oder Carbalkoxigruppen enthalten kann. $R^1$ kann auch für Alkoxi- oder Alkylthiogruppen, wie für Methoxi- oder Methylthiogruppen oder für Acyloxigruppen stehen. $R^1$ kann auch ein gegebenenfalls substituierter Arylrest sein, wie ein Phenylrest, der z.B. Alkyl- oder Alkoxigruppen oder Halogenatome enthalten kann.

Beispielsweise seien die folgenden Aldehyde der Formel III genannt : Propanal, Butanal, Pentanal, 3-Pentenal, 4-Pentenal, 3-Methyl-butanal, Phenylacetaldehyd, 3-Phenyl-propanal, 3-Phenyl-butanal, 3-Anisyl-propanal und -butanal, 3-Pyridyl-propanal, 4-Hydroxibutanal, 4-Acetoxibutanal, 5-Formylvaleriansäure, 5-Formylvaleriansäureester, 4-Dimethylamino-butanal, Methoxiacetaldehyd, Ethoxiacetaldehyd, 3-Methylthio-propanal, Acetoxiacetaldehyd, 4-Methylthio-butanal, 3,6-Dioxa-heptanal, 3,5-Dimethyloct-5-en-al, 4-Oxapentanal, 4,7-Dioxa-octanal.

Man setzt die Acetale der Formel II mit den Aldehyden der Formel III bei Temperaturen bis 150°C, vorzugsweise bei 20 bis 120°C, insbesondere bei 40 bis 100°C um. Das Molverhältnis zwischen den Ausgangsstoffen der Formeln III und II hält man zweckmäßigerweise im Bereich 1 :1 bis 2 :1. Bevorzugt sind Molverhältnisse von 1,1 bis 1,8 :1, insbesondere von 1,2 bis 1,5 :1. Andere Molverhältnisse sind jedoch auch möglich.

Nach einer besonders vorteilhaften Arbeitsweise wird das Verfahren zur Herstellung der 4-Acetale des Butendial-(1,4) in Gegenwart eines Katalysators durchgeführt, der aus einem sekundären Amin und einer Säure, vorzugsweise einer Carbonsäure, besteht. Katalysatoren der genannten Art sind z.B. Salze der Dialkylamine Dimethylamin, Diethylamin, Diisopropylamin, Di-(iso)-butylamin, Methyl-ethyl-amin, Methyl-butylamin, Ethyl-butylamin, Methyl-hydroxiethylamin, Pyrrolidin, Piperidin, Morpholin mit einer ein- bzw. mehrbasischen Säure, besonders einer Monocarbonsäure oder Dicarbonsäure, wie Essigsäure, Propionsäure, (Iso-)Buttersäure, (Iso-)-Valeriansäure, 2-Methylbuttersäure, Hexansäure, Methyl-pentan-säure, Ethylhexansäure, Isononansäure, Methoxiessigsäuree, Pivalinsäure, Methoxi-pivalinsäure, Oxalsäure, Bernsteinsäure, Glutarsäure, Adipinsäure, Hydroxibuttersäure, Apfelsäure und Hydroxipivalinsäure. Beim Einsatz einer Aldehydsäure, wie 5-Formylvaleriansäure ist die Verwendung einer zusätzlichen Carbonsäure nicht erforderlich.

Katalysatoren dieser Art werden z.B. in der EP-B-58 927 beschrieben. Bei diesen Katalysatoren ist es nicht erforderlich, daß die Amine und die Säuren in äquivalenten Mengenverhältnissen eingesetzt werden. Die zur Umsetzung eingesetzte Katalysatormenge ist ebenfalls in weiten Grenzen variabel. Zweckmäßigerweise verwendet man eine Katalysatormenge von 1 bis 150, vorzugsweise 5 bis 100, insbesondere 20 bis 100 Mol%, bezogen auf den Ausgangsstoff der Formel II. Setzt man zum Zwecke einer raschen Reaktion unter milden Bedingungen mehr als vernachlässigbare "katalytische" Mengen Katalysator (das sind z.B. Katalysatormengen von mehr als 5 Mol%, bezogen auf II) ein, so wird man den Katalysator nach Abtrennung des Produktes, z.B. durch Phasentrennung, Extraktion oder Destillation, zurückgewinnen und wiederholt einsetzen, so daß insgesamt ein geringer Verbrauch resultiert.

Nach dem neuen Verfahren erhält man die 4-Acetale des Butendial-(1,4) aus wirtschaftlich günstig zugänglichen Grundbausteinen auf verfahrenstechnisch einfach durchführbare Weise. So ermöglicht es z.B. die Herstellung des 4-Neopentylglykolacetals des Methylfumardialdehyds, indem man 2-Formyl-5,5-dimethyldioxan-(1,3) erfindungsgemäß mit Propionaldehyd entsprechend der Gleichung :

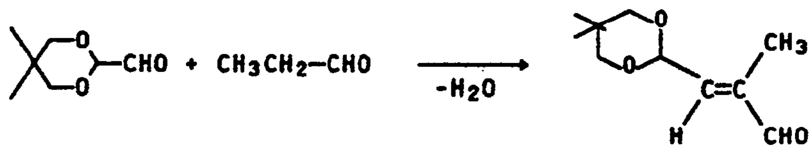

umsetzt. Überraschend war besonders die hohe Effizienz des neuen Verfahrens. Man erhält die gewünschten Verbindungen der Formel I mit hohen Ausbeuten bei gleichzeitig hoher "trans-Selektivität". Überraschenderweise spielen auch zahlreiche potentielle Konkurrenzreaktionen nur eine untergeordnete oder keine Rolle. So hätte man vorwiegend oder ausschließlich Autoaldolisierungen und Autokondensation der Ausgangsverbindungen II bzw. III sowie Coaldolisierungen erwarten können.

Das Ergebnis der erfindungsgemäßen Umsetzung ist umso überraschender, als man durch Umsetzung von Glyoxalmonoacetal mit Acetaldehyd nicht das entsprechende 4-Acetal des Fumaraldehyds, sondern höhermolekulare Produkte des Acetaldehyds erhält.

Diese Erfindung betrifft auch die neuen Acetale des Butendial-(1,4) der Formel

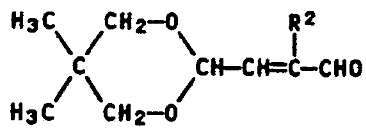 IV,

in der $R^2$ einen Alkyl-, Alkenyl- oder Alkinylrest mit 2 bis 8 C-Atomen, der Phenyl-, Pyridyl-, Hydroxi-, Alkoxi-, Thioether-, Acetoxi-, Alkylamino-, Carboxi- oder Carbalkoxigruppen enthalten kann, eine Alkoxi-, Alkylthio- oder Acyloxigruppe oder einen Phenylrest, der durch Alkyl-oder Alkoxigruppen oder Halogenatome substituiert sein kann, bedeutet.

Diese neuen Acetale sind Zwischenprodukte für die Herstellung von Wirkstoffen.

## Beispiel 1

Man erwärmt ein Gemisch aus 90g Dimethylamin in 135g Wasser und 120g Essigsäure auf 50°C, läßt bei dieser Temperatur unter Kühlen und Rühren im Verlauf einer Stunde die Lösung von 232g Propionaldehyd in 288g 2-Formyl-5,5-dimethyl-dioxan-(1,3) zulaufen, erhöht dann die Temperatur auf 80°C und rührt das Reaktionsgemisch eine Stunde weiter. Nach Abkühlen auf Raumtemperatur trennt man die wäßrige Katalysatorlösung (untere Phase) ab und erhält durch fraktionierende Destillation der organischen Phase 298g 4-Neopentylglykolacetal des 2-Methylfumardialdehyds (=2-(2-Formyl-propenyl)-5,5-dimethyldioxan-(1,3) vom Siedepunkt 68 bis 71°C/2 mbar, entsprechend 81% der Theorie, bezogen auf 2-Formyl-5,5-dimethyl-dioxan-(1,3), daneben 104g 2-Methylpentenal (Sdp. 30 bis 40°C/30 mbar).

## Beispiel 2

Man läßt in ein Gemisch aus 48g Dimethylamin in 72g Wasser und 80g Propionsäure unter Rühren bei 40°C während 30 Minuten 504g der Lösung von 240g Propionaldehyd in 264g Diethoxiacetaldehyd (Glyoxal(mono)-diethylacetal) einlaufen. Das Reaktionsgemisch wird danach noch 3 Stunden gerührt. Nach Abtrennung der unteren wäßrigen Phase erhält man durch fraktionierende Destillation der organischen Phase 237g 2-Methyl-4,4-diethoxi-but-2-en-al vom Sdp. 67 bis 69°C bei 8 mbar, entsprechend 68,9% der Theorie.

## Beispiel 3

Man läßt in ein Gemisch aus 90g Dimethylamin in 135g Wasser und 120g Essigsäure bei 80°C unter Kühlen und Rühren während 30 Minuten die Lösung von 163g Propionaldehyd in 288g 2-Formyl-5,5-dimethyl-dioxan(1,3) einlaufen. Danach wird das Reaktionsgemisch bei gleicher Temperatur noch 1 Stunde gerührt. Nach Abtrennen der unteren wäßrigen Phase erhält man durch fraktionierende Destillation der organischen Phase eine Fraktion von 40g Methylpentenal (Sdp. 34 bis 38°C/ 30 mbar, 20% bzg. auf Propionaldehyd) und 328g 4-Neopentylglykolacetal des 2-Methylfumardialdehyds vom Sdp. 70 bis 72°C bei 2 mbar, entsprechend 89,1% d.Th. sowie eine Fraktion von 18 g 2-(1-Hydroxi-2-formylpropyl)-5,5-dimethyldioxan (Aldol), entsprechend 4,45% d.Th.

Beispiel 4

Entsprechend Beispiel 3 setzt man die Lösung von 116g Propionaldehyd in 288g 2-Formyl-5,5-dimethyl-dioxan-(1,3) um und erhält 356g organische Phase, die nach gaschromatographischer Analyse enthält: 3,1 Gew.% Methylpentenal, 2,7 Gew.% 2-Formyl-5,5-dimethyl-dioxan-(1,3), 5,8 Gew.% 2-(1-Hydroxi-2-formyl-propyl)-5,5-dimethyl-dioxan-(1,3) und 86,8 Gew.% 4-Neopentylglykolacetal des 2-Methyl-fumardialdehyds, entsprechend 84% d.Th.

Beispiel 5

Entsprechend Beispiel 3 setzt man die Lösung von 288g n-Butyraldehyd in 288g 2-Formyl-5,5-dimethyl-dioxan-(1,3) um und erhält durch fraktionierende Destillation 285,6g 4-Neopentylglykolacetal des 2-Ethyl-fumardialdehyds vom Siedebereich 82 bis 84°C/1 mbar, entsprechend 72,1% d.Th., daneben als Vorlauf 128g 2-Ethylhexenal.

Beispiel 6

Entsprechend Beispiel 3 setzt man die Lösung von 300 g Phenylacetaldehyd in 288g 2-Formyl-5,5-dimethyl-dioxan-(1,3) um und erhält durch fraktionierende Destillation 331g 4-Neopentylglykolacetal des 2-Phenylfumardialdehyds vom Siedebereich 124 bis 126°C/1 mbar, entsprechend 67,2% d.Th.

Beispiel 7

Entsprechend Beispiel 3 setzt man die Lösung von 415g 3-Phenylbutanal in 288g 2-Formyl-5,5-dimethyl-dioxan-(1,3) um und erhält durch fraktionierende Destillation 490g 4-Neopentylglykolacetale des 2-(1-Phenyl-ethyl)-buten-2-dials (1,4) (E/Z = 2/1) vom Siedebereich 160 bis 162°C/1 mbar, entsprechend 65,5% d.Th.

Beispiel 8

Man erwärmt ein Gemisch aus 129g Dibutylamin und 74g Propionsäure auf 60°C und läßt bei dieser Temperatur unter Rühren und Kühlen im Verlauf von 30 Minuten das Gemisch aus je 144g 2-Formyl-5,5-dimethyldioxan-(1,3)und 5-Formyl-valeriansäuremethylester zulaufen. Man läßt weitere 2 Stunden rühren, kühlt auf Raumtemperatur ab, fügt unter Rühren 400 ml Wasser zu, trennt die organische Phase ab und wäscht sie 3 mal mit je 150 ml Wasser. Durch fraktionierende Destillation gewinnt man 172,3g 4-Neopentylglykolacetal des 2(3-Methoxicarbonyl)propyl-buten-2-dials-1,4 (E/Z = 20/1) vom Siedebereich 148 bis 154°C/2 mbar, entsprechend 63,8% d.Th.

## Ansprüche

1. Verfahren zur Herstellung von 4-Acetalen des Butendial-(1,4) der Formel

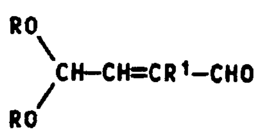

I

in der R für einen Alkyl-, Alkenyl-, Cycloalkyl- oder Aralkylrest mit 1 bis 12 C-Atomen steht, der Alkoxygruppen enthalten kann, oder beide R zusammen einen Alkylen- oder Alkenylenrest mit 2 bis 10 C-Atomen, der Alkoxigruppen enthalten kann, bedeuten und $R^1$ einen Alkyl-, Alkenyl- oder Alkinylrest mit 1 bis 12 C-Atomen, der durch cycloaliphatische, aromatische oder heterocyclische Reste oder durch Hydroxi-, Ether-, Thioether-, Acyl-, Alkylamino-, Carboxi- oder Carbalkoxygruppen substituiert sein kann, einen gegebenenfalls substituierten Arylrest oder eine Alkoxi-, Alkylthio- oder Acyloxygruppe bedeutet, dadurch gekennzeichnet, daß man Glyoxalmonoacetale der Formel

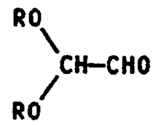 II,

in der die Reste R die oben genannte Bedeutung haben, mit Aldehyden der Formel

$R^1-CH_2-CHO$ III

in der $R^1$ die obengenannte Bedeutung hat, bei Temperaturen von 20 bis 150°C umsetzt.

2. Verfahren nach Anspruch 1, dadurch gekennzeichnet, daß man die Umsetzung in Gegenwart eines sekundären Amins und einer Säure vornimmt.

3. Verfahren nach Anspruch 2, dadurch gekennzeichnet, daß man als Säure eine Carbonsäure verwendet.

4. Acetale des Butendial-(1,4) der Formel

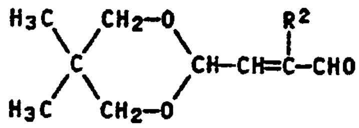 IV,

in der $R^2$ einen Alkyl-, Alkenyl- oder Alkinylrest mit 2 bis 8 C-Atomen, der Phenyl-, Pyridyl-, Hydroxi-, Alkoxi-, Thioether-, Acetoxi-, Alkylamino-, Carboxi- oder Carbalkoxigruppen enthalten kann, eine Alkoxi-, Alkylthio- oder Acyloxigruppe oder einen Phenylrest, der durch Alkyl-oder Alkoxigruppen oder Halogenatome substituiert sein kann, bedeutet.

5. Acetale des Butendial-(1,4) der Formel

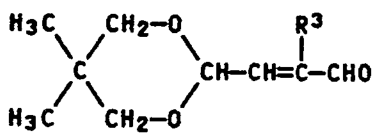 V

in der $R^3$ einen der Reste

$-CH_2-CH_3$, $-CH_2-CH_2-CH_2-COOCH_3$, 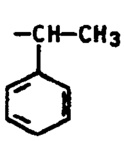 oder 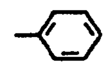

bedeutet.

## Claims

1. A process for the preparation of a 4-acetal of butene-1,4-dial of the formula

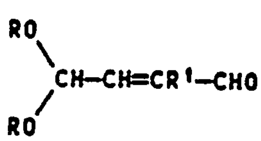 I

where R is an alkyl, alkenyl, cycloalkyl or aralkyl radical of 1 to 12 carbon atoms which may contain alkoxy groups, or the two radicals R together form an alkylene or alkenylene radical of 2 to 10 carbon atoms which

may contain alkoxy groups, and $R^1$ is an alkyl, alkenyl or alkynyl radical of 1 to 12 carbon atoms which may be substituted by cycloaliphatic, aromatic or heterocyclic radicals or by hydroxyl, ether, thioether, acyl, alkylamino, carboxyl or carbalkoxy groups, or is an unsubstituted or substituted aryl radical or an alkoxy, alkylthio or acyloxy group, wherein a glyoxal monoacetal of the formula

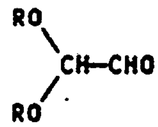
II,

where R has the above meanings, is reacted with an aldehyde of the formula

$$R^1\text{-}CH_2\text{-}CHO \quad III$$

where $R^1$ has the above meanings, at from 20 to 150°C.

2. A process as claimed in claim 1, wherein the reaction is carried out in the presence of a secondary amine and of an acid.

3. A process as claimed in claim 2, wherein the acid used is a carboxylic acid.

4. An acetal of butene-1,4-dial of the formula

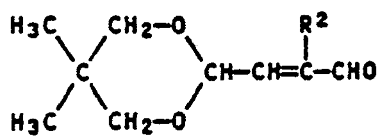
IV,

where $R^2$ is an alkyl, alkenyl or alkynyl radical of 2 to 8 carbon atoms which may contain phenyl, pyridyl, hydroxyl, alkoxy, thioether, acetoxy, alkylamino, carboxyl or carbalkoxy groups, or is alkoxy, alkylthio or acyloxy or a phenyl radical which may be substituted by alkyl, alkoxy or halogen.

5. An acetal of butene-1,4-dial of the formula

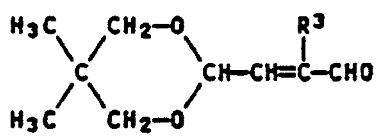
V

where $R^3$ is one of the radicals

$-CH_2-CH_3-$, $-CH_2-CH_2-CH_2-COOCH_3-$, $-CH-CH_3$ 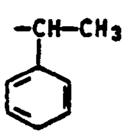 or 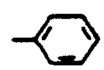

## Revendications

1. Procédé de préparation de 4-acétals du butène-1,4-dial de formule

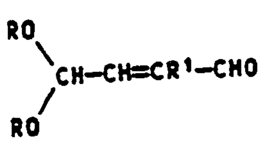
I

dans laquelle R est mis pour un groupement alkyle, alcényle, cycloalkyle ou aralkyle ayant de 1 à 12 atomes de carbone, qui peut contenir des groupements alcoxy, ou les deux restes R ensemble représentent un reste alkylène ou alcénylène de 2 à 10 atomes de carbone qui peut contenir des groupements alcoxy, et R[1] représente un reste alkyle, alcényle ou alcynyle ayant de 1 à 12 atomes de carbone qui peut être substitué par des restes cycloaliphatiques, aromatiques ou hétérocycliques ou par des groupements hydroxy, éther, thioéther, acyle, alkylamino, carboxy ou carbalcoxy, un reste aryle éventuellement substitué ou un groupement alcoxy, alkylthio ou acyloxy, caractérisé en ce qu'on fait réagir à des températures de 20 à 150°C des monoacétals de glyoxal de formule

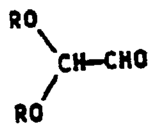 II,

dans laquelle les restes R ont la signification donnée ci-dessus, avec des aldéhydes de formule

$R^1-CH_2-CHO$ III

dans laquelle R[1] a la signification donnée ci-dessus.

2. Procédé selon la revendication 1, caractérisé en ce qu'on effectue la réaction en présence d'une amine secondaire et d'un acide.

3. Procédé selon la revendication 2, caractérisé en ce qu'on utilise comme acide un acide carboxylique.

4. Acétals de butènel-1,4-dial de formule

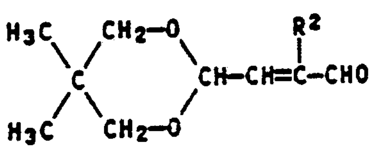 IV,

dans laquelle R[2] représente un reste alkyle, alcényle ou alcynyle ayant de 2 à 8 atomes de carbone qui peut contenir des groupements phényle, pyridyle, hydroxy, alcoxy, thioéther, acétoxy, alkylamino, carboxy ou carbalcoxy, un reste alcoxy, alkylthio ou acyloxy ou un reste phényle qui peut être substitué par des groupements alkyle ou alcoxy ou des atomes d'halogène.

5. Acétals de butènel-1,4-dial de formule

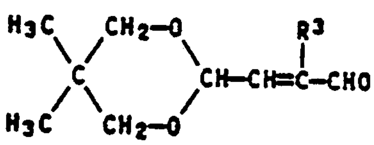 V

dans laquelle R[3] représente l'un des restes

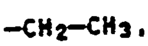, $-CH_2-CH_2-CH_2-COOCH_3$, 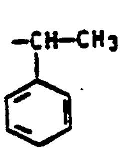 ou 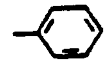.